(19) Europäisches Patentamt
European Patent Office
Office européen des brevets

(11) Publication number: 0 064 384 B1

(12) EUROPEAN PATENT SPECIFICATION

(45) Date of publication of patent specification: 23.07.86

(51) Int. Cl.[4]: C 07 C 143/11, E 21 B 43/22

(21) Application number: 82302170.4

(22) Date of filing: 28.04.82

# (54) Alkoxypolyethoxypropane sulfonates, process for their preparation and method for their use in enhanced oil recovery.

(30) Priority: 30.04.81 US 259216
30.04.81 US 259215

(43) Date of publication of application:
10.11.82 Bulletin 82/45

(45) Publication of the grant of the patent:
23.07.86 Bulletin 86/30

(84) Designated Contracting States:
BE DE FR GB IT NL

(56) References cited:
FR-A-2 095 772
FR-A-2 353 527
GB-A- 719 445

(73) Proprietor: MOBIL OIL CORPORATION
150 East 42nd Street
New York New York 10017 (US)

(72) Inventor: Hsia Chen, Catherine Shuihua
102 Pine Grove Road
Berkeley Heights New Jersey (US)
Inventor: Williams, Albert Lloyd
9 Batberry Road, R.D.2
Princeton New Jersey (US)

(74) Representative: West, Alan Harry et al
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 064 384 B1

**Description**

This invention relates to alkoxypolyethoxypropane sulfonates and to an oil recovery process using them.

In the recovery of oil from oil-bearing deposits, it is generally possible to recover only a portion of the original oil by so called "primary methods" which utilize only the natural forces present in the reservoir or deposit. A variety of supplemental techniques have therefore been employed in order to increase the recovery of oil from these subterranean reservoirs. The most widely used supplemental recovery technique is water flooding which involves injection of water into an oil bearing reservoir. However, there are problems associated with the water flooding technique and water-soluble surfactants have generally been required to be used for this process to be completely successful. The LTWF (Low Tension Water Flood) method using surfactants which function in conditions of low salinity (less than 3 percent) is well known but it has been found that preflushing the reservoirs with fresh or low salinity water to reduce the salinity so that the low salinity surfactants may be used is not always effective, or that preflushing is effective only for a short time and the salinity of the fresh water increases over a period of time since it is in contact with reservoir rocks and clays. Either event renders the low salinity surfactants useless and therefore it is of vital importance to have a surfactant which functions at the salinity of the reservoir water to negate the necessity of preflushing.

Two different concepts have been developed for using surfactants to enhance oil recovery. In the first, a solution containing a low concentration of surfactants is injected into the reservoir. The surfactant is dissolved in either water or oil and is in equilibrium with surfactant aggregates known as micelles. Large pore volumes (about 15—60% or more) of the solution are injected into the reservoir to reduce interfacial tension between oil and water and thereby increase oil recovery. Specific relationships exist between interfacial tensions of the oil against the flooding media and the percentage of recovery obtained by flooding, i.e., the efficiency of flooding increases as the interfacial tension decreases. Oil may be banked with the surfactant solution process but residual oil saturation at a given position in the reservoir will only approach zero after passage of large volumes of surfactant solution.

In the second process, a relatively small pore volume (about 3—20%) of a higher concentration surfactant solution is injected into the reservoir. With the higher surfactant concentration, the micelles become a surfactant-stabilized dispersion of either water and hydrocarbon or hydrocarbon and water. The high surfactant concentrations allow the amount of dispersed phase in the microemulsion to be high in comparison to the dispersed phase of the micelles in the low concentration surfactant solutions.

The injected solution (slug) is formulated with three or more components. The basic components (hydrocarbon, surfactant, and water) are sufficient to form the micellar solutions. A fourth, co-surfactant component (usually alcohol) can be added. Electrolytes, normally inorganic salts form a fifth component that may be used in preparing the micellar solutions of microemulsions. The high concentration surfactant solutions displace both oil and water and readily displace all the oil contacted in the reservoir. However, as the high concentration slug moves in the reservoir, it is diluted by formation flood and the process reverts to a low concentration flood (Enhanced Oil Recovery, Van Poolen & Associates, 1980, Tulsa, Oklahoma).

Work is still in progress in the laboratory and in the field to select the optimum method of injecting surfactant to improve oil recovery. The best process for a specific reservoir is the one having the potential to provide the greatest efficiency and yield regardless of the concentration of the surfactant. However, to be efficient the chemical system must be tailored to the specific reservoir.

The prior art with respect to the use of surfactant polymer floods to recover oil from reservoirs discloses that for a given amount of surfactant, a small slug process with a high surfactant concentration is more efficient than a large slug process with a low surfactant concentration. The former produces oil earlier but takes a smaller number of pore volumes to complete oil production. This is a favorable condition. However, it has become evident that fluid dispersion and mixing take place in the reservoirs and the slug intake routine cannot be maintained. Deterioration of the surfactant and the mobility control slug can lead to process failure or at least a reduction in process efficiency. For heterogeneous reservoirs where fluid dispersion and mixing take place to a greater extent it is desirable if not vital to have a continuous flooding process with a surfactant that is capable of moving even at very low concentrations.

Propane sulfonates of various polyethoxylated alcohols are known to be effective as surfactants and various propane sulfonates are known to exhibit a high tolerance towards brine and to possess high thermal and hydrolytic stabilities.

The present invention is based on the observation that certain propane sulfonate surfactants can be used alone in low concentrations over a wide range of salinity conditions in continuous flooding techniques to increase oil production during secondary water flooding or to recover tertiary oil from reservoirs that have already been water-flooded.

According to the present invention, there are provided compounds of the general formula

$$R_1—\underset{\substack{|\\ R_2}}{CH}—CH_2—O(C_2H_4O)_xCH_2CH_2CH_2SO_3Na$$

in which $R_1$ is an alkyl group having from 6 to 26 carbon atoms, $R_2$ is an alkyl group having from 2 to 10 carbon atoms, and *x* has a value from 2 to 6, preferably 3.

Such alkoxypolyethoxypropanesulfonates have been found to be useful in enhanced oil recovery, especially when used at low concentrations, and to be especially adaptable to high salinity oil reservoirs, that is to say reservoirs having a salinity of from 4 to 30%. Such compounds can be used as single component surfactants (without the addition of any other component or co-surfactant) although it may be desirable to use a mixture of two or more such compounds or to use the surfactant in combination with a sacrificial compound such as lignin sulfonate.

The present invention therefore also provides a method for recovering oil from a subterranean oil reservoir having one or more production wells and one or more injection wells in contact with the reservoir comprising injecting into the oil reservoir an aqueous medium containing as the sole surfactant a branched alkoxypolyethoxypropanesulfonate as described above in a concentration sufficient to increase oil production from the production well during secondary water flooding or to recover residual tertiary oil when the oil reservoir has been previously flooded.

Surfactants according to the invention in which $R_1$ is $C_6$-alkyl and $R_2$ is $C_4$-alkyl are highly effective in reservoirs having a salinity from about 15 to about 28 weight percent. When $R_1$ is $C_7$-alkyl and $R_2$ is $C_5$-alkyl, the surfactants are very effective in salinities of from about 10 to 20 percent, and when $R_1$ is $C_8$-alkyl and $R_2$ is $C_6$-alkyl the surfactants are highly effective in salinities of from about 4 to 10 percent.

The branched sulfonates of the invention generally have their minimum interfacial tension at salinities of less than about 30%, typically at about 4 to 28%. Indeed, the surfactants can be tailored for use in particular salinity ranges by varying the overall chain length of the branched alkyl radical and the length of the branches. In contrast to the branched surfactants of the invention whose optimum (minimum) interfacial tension occurs at salinities below about 30%, the corresponding straight chain alkyl surfactants of the prior art with the same total number of carbon atoms exhibit minimum interfacial tension at 30% salinity or higher. A consequence of this is that the branched surfactants exhibit significantly lower interfacial tension than the corresponding straight chain surfactants at salinities of most interest in chemical water flooding, that is at salinities of 30% or less. In general, at salinities of less than 30% the branched surfactants have interfacial tension values of a factor of 0.5 or less than the corresponding straight chain surfactant containing the same number of carbon atoms at the same surfactant concentration. Typically, as shown in the following Examples, the factor is much less than 0.5, rendering the branched surfactants of this invention particularly suitable for use in water flooding.

The compounds according to the invention can be prepared by a number of different processes. For example, the literature describes the preparation of propane sulfonates of alcohols by the reaction of alkali metal salts of the alcohols with propane sultone:

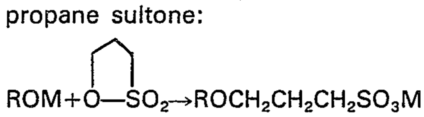

This is a convenient, high-yield synthesis but is not appropriate on a large scale for several reasons, especially since such a reaction requires multistep synthesis and purification of propane sultone; propane sultone is expensive to purify and its overall yield of 80—90% limits the yield in the preparation of propane sulfonates; and propane sultone is a known carcinogen. Processes involving the use of propane sultone must therefore incorporate expensive safety features but will always engender some risk.

The literature also describes a two-step process for the preparation of propane sulfonates of tertiary amines [*J. Amer. Oil Chem. Soc.*, 53, 60 (1976)] but reports that the process produces excessive quantities of undesirable "iso-sulfonate" whose presence degrades the performance of the product. This, of course, is most undesirable.

The preparation of allyl ethers by the reaction of sodium or sodium methoxide with the alcohol followed by reaction with allyl chloride all in an organic solvent such as toluene or tetrahydrofuran (the Williamson ether synthesis) is well known and may be found in many standard textbooks on organic chemistry. The reaction of allyl chlorides and alcohols with NaOH is less well known but does occur in the presence of appropriate phase transfer catalysts. Hitherto, such catalysts have invariably been tetraalkylammonium salts. Thus H. H. Freedman in *Tetrahedron Letters*, 3251 (1975) reports that the reaction of allyl alcohol with benzyl chloride may be achieved with NaOH as base if benzyl triethylammonium chloride is used as a phase transfer catalyst. The yield is only 72%. If such a tetraalkylammonium chloride is used, it must be separated from the product and recycled in order that it does not contaminate the product or represent a loss of costly material. Such a process is therefore expensive to carry out.

The reaction of $NaHSO_3$ with simple olefins has been much studied. The literature teaches that for simple water-soluble olefins or olefins that can be made soluble by the addition of small amounts of alcohols, all that is required for high conversion into the desired product is conditions in which all reagents are dissolved in a single phase.

However, the early literature on the reaction of $NaHSO_3$ with olefins indicates that S-alkylation leading to sulfonate products might be accompanied by a slight amount of O-alkylation leading to sulfites but even this slight formation is disputed and the literature for the last thirty years reveals that sulfites are actually

not by-products of the sulfitation of olefins. It was therefore quite surprising to find in developing the present invention that sulfitation of allyl ethers can produce as much as 100% sulfite in solvents which are taught in the literature to be acceptable for sulfitation of ordinary olefins to sulfonates. A high yield of sulfite is also surprising because the literature teaches that solvent systems which produce high conversions of olefins will product high yields of sulfonates.

The compounds according to the invention are therefore advantageously prepared by a process which comprises reacting together an alcohol $RO(C_2H_4O)_xH$, sodium hydroxide and allyl halide or tosylate to form the corresponding allyl ether $RO(C_2H_4O)_xCH_2CH=CH_2$ and reacting the allyl ether with sodium bisulfite, the first stage of the reaction being carried out in approximately 50% for example 30—70%, especially 40 to 60%, aqueous sodium hydroxide in the presence of sufficient final product to function as a phase transfer catalyst, and the second stage being carried out without isolation of the intermediate allyl ether.

This process has several inherent advantages over known processes for the production of propane sulfonates. Firstly, it uses aqueous sodium hydroxide as opposed to the sodium or sodium alcoholate and anhydrous conditions previously employed; secondly, it eliminates the use, and hence the cost of separation, recovery and recycle of organic solvent; thirdly, since intermediate product isolation is unnecessary, the process can be carried out in a single reaction vessel; and fourthly, the product is obtained in a high yield—rarely below 90%—compared to yields of 40 to 50% for many known processes.

The two-stage process can be represented schematically as follows:

$$RO(C_2H_4O)_xH+NaOH+XCH_2CH=CH_2 \rightarrow RO(C_2H_4O)_xCH_2CH=CH_2+NaX$$

$$RO(C_2H_4O)CH_2CH=CH_2+NaHSO_3 \rightarrow RO(C_2H_4O)_xCH_2CH_2CH_2SO_3Na$$

R being the group $R_1(R_2)CHCH_2$, *x* having the meaning given above and X being halide or tosylate. The reaction is suitable carried out at 0 to 55°C at ambient pressure in the presence of from 0.1 to 1 mole of final product per mole of alcohol $RO(C_2H_4O)_xH$. Any excess, unreacted allyl compound $XCH_2CH=CH_2$ may be distilled off at the end of the reaction.

Alternatively, of course, the compounds of the invention can be prepared by the method which is in itself known for the preparation of substituted propane sulfonates, by the reaction of an alkali metal salt of the alcohol $RO(C_2H_4O)_xH$ with propane sultone.

The following Examples illustrate the invention.

Example 1

Preparation of branched dodecyloxytriethoxypropane sulfonate

$$C_6H_{13}—CH—CH_2—O—(C_2H_4O)_3—CH_2—CH_2—CH_2—SO_3Na$$

$$\quad |$$

$$C_4H_9$$

2-Butyloctanol was reacted with an equivalent amount of phosphorous tribromide at substantially 0°C. The resulting 2-butylbromooctane was purified by vacuum distillation. The distilled 2-butylbromooctane was ethoxylated by reacting with excess triethylene glycol. One equivalent of metallic sodium was first reacted with the triethylene glycol in tetrahydrofuran. This was followed by the addition of the bromo-compound and the reaction mixture was then refluxed for 4 days. The triethoxylated alcohol product was purified by vacuum distillation and the purity was checked by GC. The purified triethoxylated alcohol was reacted with 0.98 equivalent of metallic sodium in toluene followed by one equivalent of distilled propane sultone in toluene. The final product was purified by liquid chromatography. The purity of the final product was established by NMR spectroscopy.

The interfacial tension of this product in aqueous solutions of various concentrations and of various salinities was then determined, against a crude oil.

A spinning drop Interfacial Tensionmeter was used, and measurements were made after 30 minutes spinning at 10 or 14 msec., or longer, or until no more change in the drop took place. The width of the drop was then measured. The interfacial tension was calculated according to the equation:

$$IFT=\frac{9.8696\times10^6\times\Delta d\times\left(\frac{Dm}{66.6}\right)}{p^2}$$

where

IFT=Interfacial Tension, dynes/cm

Δd=difference in density between oil and brine, g/ml

Dm=diameter of oil drop, mm

P=spinning speed, msec

The data obtained is set out in Table 1.

TABLE 1

| Salinity, percent | Concentration, percent | IFT, dyne/cm |
|---|---|---|
| 16.6 | 2 | 0.019 |
| | 1 | 0.021 |
| | 0.5 | 0.024 |
| | 0.1 | 0.021 |
| | 0.01 | 0.016 |
| | 0.001 | 0.002 |
| 20.0 | 2 | 0.019 |
| | 1 | 0.022 |
| | 0.5 | 0.011 |
| | 0.1 | 0.008 |
| | 0.01 | 0.007 |
| | 0.001 | 0.006 |
| 26.0 | 2 | 0.005 |
| | 1 | 0.003 |
| | 0.5 | 0.017 |
| | 0.1 | 0.010 |
| | 0.01 | 0.042 |
| | 0.001 | 0.041 |

Surprisingly low tensions persisted over a span of salinity of 10 percent, and over a concentration range of three decades.

Example 2 (comparative)
Preparation of linear dodecyloxytriethoxypropane sulfonate

$$n—C_{12}H_{25}—O—(C_2H_4O)_3—CH_2—CH_2—CH_2—SO_3Na$$

The procedure described in Example 1 was used except that 1-dodecanol was used in place of 2-butyloctanol. The interfacial tension data for the sulfonate were obtained in the manner described in Example 1 and are listed in Table 2.

TABLE 2

| Salinity, percent | Concentration, percent | IFT, dyne/cm |
|---|---|---|
| 16.6 | 2 | 0.21 |
| | 1 | 0.180 |
| | 0.5 | 0.193 |
| | 0.1 | 0.162 |
| | 0.01 | 0.140 |
| | 0.001 | 0.117 |
| 20.0 | 2 | 0.148 |
| | 1 | 0.164 |
| | 0.5 | 0.144 |
| | 0.1 | 0.150 |
| | 0.01 | 0.157 |
| | 0.001 | 0.066 |
| 26.0 | 2 | 0.085 |
| | 1 | 0.094 |
| | 0.5 | 0.066 |
| | 0.1 | 0.077 |
| | 0.01 | 0.049 |
| | 0.001 | 0.069 |

Clearly the compound of Example 2 does not produce ultra-low interfacial tension between oil and water in the specified salinity range.

Example 3
Preparation of branched tetradecyloxytriethoxypropane sulfonate

$$C_7H_{15}—CH—CH_2—O—(C_2H_4O)_3—CH_2—CH_2—CH_2—SO_3Na$$
$$|$$
$$C_5H_{11}$$

The procedure described in Example 1 was used except that in place of 2-butyloctanol, an equivalent amount of 2-pentylnonanol was used. The interfacial tension data listed in Table 3 were obtained as described in Example 1.

TABLE 3

| Salinity, percent | Concentration, percent | IFT, dyne/cm |
|---|---|---|
| 10.0 | 2 | 0.019 |
| | 1 | 0.023 |
| | 0.5 | 0.027 |
| | 0.1 | 0.015 |
| | 0.01 | 0.006 |
| | 0.001 | 0.013 |
| 14.4 | 2 | 0.009 |
| | 1 | 0.008 |
| | 0.5 | 0.006 |
| | 0.1 | 0.007 |
| | 0.01 | 0.012 |
| | 0.001 | 0.055 |
| 16.6 | 2 | 0.009 |
| | 1 | 0.008 |
| | 0.5 | 0.013 |
| | 0.1 | 0.018 |
| | 0.01 | 0.037 |
| | 0.001 | 0.096 |

This $C_{14}$-alkyl compound produces low tension at lower salinities than the $C_{12}$-alkyl compound of Example 1.

Example 4
Preparation of branched hexadecyloxytriethoxypropane sulfonate

$$C_8H_{17}—CH—CH_2—O—(C_2H_4O)_3—CH_2—CH_2—CH_2—SO_3Na$$
$$|$$
$$C_6H_{13}$$

The procedure described in Example 1 was used except that in place of 2-butyloctanol, an equivalent amount of 2-hexyldecanol was used. The interfacial tesion data of the product are listed in Table 4.

TABLE 4

| Salinity, percent | Concentration, percent | IFT, dyne/cm |
|---|---|---|
| 10.0 | 1 | 0.012 |
| | 0.1 | 0.026 |
| | 0.01 | 0.040 |
| | 0.005 | 0.019 |
| 8.0 | 1 | 0.007 |
| | 0.1 | 0.007 |
| | 0.01 | 0.005 |
| 6.0 | 1 | 0.007 |
| | 0.1 | 0.005 |
| | 0.01 | 0.006 |
| | 0.005 | 0.008 |
| 4.0 | 1 | 0.019 |
| | 0.1 | 0.032 |
| | 0.01 | 0.014 |
| | 0.005 | 0.014 |
| 2.0 | 1 | 0.039 |
| | 0.1 | 0.044 |
| | 0.01 | 0.030 |
| | 0.001 | 0.033 |

Example 5

155.9 grams of Berea sand (less than 325 mesh) were packed into a 1.8 m glass column. The pore volume of this sand-pack column was determined to be 35 ml (by vacuum filling the dry column with West Burkburnett (WBB) brine having 16.6 percent salinity). The brine was displaced by crude oil until no more brine was produced. The crude oil in the column was equivalent to the oil in a reservoir before secondary water flooding. The column was then flooded with the 16.6 percent brine until no more oil was produced. This was, accordingly, equivalent to secondary water flooding in the field. The oil which remained in the column was equivalent to tertiary oil in the field and was determined to be 9.2 ml or 26.9 percent saturation.

Into the column containing the tertiary oil a slug of 0.2 pore volume (or 7 ml) of a 2 percent solution of the sulfonate prepared in Example 1 in 16.6 percent brine was injected; nothing else was added. This slug of surfactant was followed by addition of a mobility control slug consisting of 0.1 percent Xanthan gum in 1 percent aqueous NaCl solution until no more oil was produced. By this process oil started to produce when 0.87 pore volume of fluid was pumped through; 43.5 percent oil was produced after 2 pore volumes and 45 percent was produced after 3.58 pore volumes.

This clearly shows that the branched alkoxypolyethoxypropane sulfonate is suitable for use alone to enhance tertiary oil recovery without the need of a co-surfactant, even under high brine conditions.

Example 6

An oil recovery experiment similar to that described in Example 5 was performed, except that a 20 percent brine solution was used instead of a 16.6 percent solution. The results are given below:

| | |
|---|---|
| Weight of sand: | 155.8 gm |
| Pore volume: | 36.0 ml |
| Tertiary oil: | 9.7 ml |
| Surfactant slug: | 1.2 pv of 2 percent surfactant in 20 percent WBB brine |
| Mobility control slug: | 0.1 percent Xanthan gum in 1 percent NaCl |
| Oil recovery: | Started at 0.7 pv; 13.4 percent at 0.75 pv; 46.4 percent at 1.34 pv; 47.9 percent at 1.83 pv. |

This data further shows that the alkoxytriethoxypropane sulfonates of the invention are suitable as single component surfactants in the recovery of tertiary oil under extremely high brine conditions.

Example 7

A 1.8 m sand-pack column containing 158.8 gm Berea sand was vacuum filled with 20% brine. The pore volume was determined to be 37.2 ml. The brine was displaced by crude oil until no more brine was produced. The oil in the column was then flooded with 20% brine until no more oil was produced leaving a

residual oil of 9.85 ml or 26.5% residual oil saturation. The column was then flooded continuously with 0.01% wt.% of the compound prepared in Example 1, in 20% brine.

Oil started to produce after 0.9 pore volume; an amount of 5.2% was produced after 1.7 pv and 9% after 3.5 pv. The process is slow but incremental oil was produced.

Example 8

The procedure described in Example 7 was repeated except that the surfactant solution also contained 3% lignin sulfonate. 16% Oil was produced after 3.5 pore volume.

Example 9

The procedure described in Example 7 was repeated except that the surfactant solution was 0.1% of the compound of Example 1 in 20% brine. A recovery of 12% was achieved after 4 pore volumes.

Example 10

The procedure described in Example 9 was repeated except that the surfactant solution also contained 3% lignin sulfonate. A recovery of 50% residual oil was achieved after 3 pore volumes and 80% after 4 pore volumes. This experiment shows that lignin sulfonate significantly improves the oil recovery.

Example 11

The procedure described in Example 10 was repeated except that the surfactant solution contained 1% lignin sulfonate. A recovery of 50% was achieved after 4 pore volumes and 75% after 5 pore volumes.

Example 12

The procedure described in Example 7 was repeated except that the 20% brine was replaced by 16.6% brine. The surfactant solution contained 0.1 wt.% of the compound of Example 1 and 1 wt.% lignin sulfonate as a sacrificial chemical. The column was flooded by this surfactant solution in a continuous mode; 37% tertiary oil was recovered after 2.34 pore volume and 60% after 3.74 pore volume.

Example 13

The procedure described in Example 7 was repeated except that 12% brine was used, the surfactant solution contained 0.1 wt.% of the compound of Example 3, and 1 wt.% lignin sulfonate. The solution was injected in a continuous mode; 11.8% tertiary oil was recovered after 3.82 pore volumes, 41.2% after 5.00 and 54.1% after 6.74 pore volumes.

Example 14

The procedure described in Example 7 was repeated except 6% brine was used, the surfactant solution contained 0.1 wt.% of the compound of Example 4, and 1% lignin sulfonate. One pore volume of this surfactant solution was injected followed by a continuous injection of 0.15% polytranpolysaccharide in 6% brine. The tertiary oil was completely recovered after 1.2 pore volume of flooding.

Example 15

This example shows that incorporation of a mobility control agent results in much earlier oil production. The sand-pack with residual oil was prepared as described in Example 7. Thereafter, a surfactant solution containing 0.3 wt.% of the compound of Example 4, 1.0 wt.% lignin sulfonate as a sacrificial chemical, and 500 ppm Polytran (a nonionic polysaccharide) in 6% brine was flooded into the sand-pack column. After 1 pore volume of the surfactant solution was flooded through tapered mobility control solutions were injected; 0.3 pore volume of 500 ppm, 0.2 pore volume of 250 ppm of Polytran in 1% NaCl followed by 1% NaCl solution. A recovery of 45% of oil was accomplished after one pore volume and 92% after 2.4 pore volume production. No pressure build up was observed.

Example 16

Similar results to those of Example 15 were obtained when the mobility control slugs were in 6% brine instead of 1% NaCl.

Example 17

Similar results to those of Example 15 were obtained when the compound of Example 1 was used in 22% brine.

Example 18

Similar results to those of Example 15 were obtained when the compound of Example 3 was used in 12% brine.

## Claims

1. A compound having the general formula

$$R_1—CH(R_2)—CH_2—O(C_2H_4O)_xCH_2CH_2CH_2SO_3Na$$

in which $R_1$ is an alkyl group having from 6 to 26 carbon atoms, $R_2$ is an alkyl group having from 2 to 10 carbon atoms, and $x$ has a value from 2 to 6.

2. A compound according to Claim 1, in which $R_1$ has from 8 to 12 carbon atoms and $R_2$ has from 4 to 6 carbon atoms.

3. A method for recovering oil from a subterranean oil reservoir having one or more production wells and one or more injection wells in contact with the reservoir, comprising injecting into the oil reservoir an aqueous medium which includes as the sole surfactant, a compound according to Claim 1 or Claim 2 in concentration effective to increase oil production from the production well during secondary water flooding or to recover residual tertiary oil when the oil reservoir has been previously flooded.

4. A method according to Claim 3, in which the oil reservoir is a high salinity reservoir.

5. A method according to Claim 4, in which the oil reservoir has a salinity of from 4 to 30% brine.

6. A method according to any one of Claims 3 to 5, in which the aqueous medium includes a sacrificial compound in an amount effective to substantially reduce adsorption of the surfactant and thereby further increase oil production from the production well.

7. A method according to Claim 6, in which the sacrificial compound is lignin sulfonate.

8. A method according to Claim 7, in which the amount of lignin sulfonate is from 0.75 to 2 weight percent.

## Patentansprüche

1. Verbindung der allgemeinen Formel

$$R_1—CH(R_2)—CH_2—O(C_2H_4O)_xCH_2CH_2CH_2SO_3Na,$$

in der $R_1$ einen Alkylrest mit 6 bis 26 Kohlenstoffatomen, $R_2$ einen Alkylrest mit 2 bis 10 Kohlenstoffatomen und x einen Wert von 2 bis 6 bedeuten.

2. Verbindung nach Anspruch 1, worin $R_1$ 8 bis 12 Kohlenstoffatome und $R_2$ 4 bis 6 Kohlenstoffatome aufweisen.

3. Verfahren zum Gewinnen von Öl aus einem unterirdischen Ölreservoir, das eine oder mehrere Produktionsbohrungen und eine oder mehrere Injektionsbohrungen im Kontakt mit dem Reservoir aufweist, dadurch gekennzeichnet, daß in das Ölreservoir ein wäßriges Medium eingespritzt wird, das als einzigen oberflächenaktiven Stoff eine Verbindung nach Anspruch 1 oder 2 in einer Konzentration enthält, die wirksam ist, um die Ölproduktion aus der Produktionsbohrung während des sekundären Flutens mit Wasser zu erhöhen, oder restliches tertiäres Öl zu gewinnen, wenn das Ölreservoir vorher geflutet worden ist.

4. Verfahren nach Anspruch 3, worin das Ölreservoir einen hohen Salzgehalt aufweist.

5. Verfahren nach Anspruch 4, worin das Ölreservoir einen Salzgehalt von 4 bis 30% Salzsole aufweist.

6. Verfahren nach einem der Ansprüche 3 bis 5, worin das wäßrige Medium eine Opferverbindung in einer Menge enthält, die wirksam ist, um die Adsorption des oberflächenaktiven Stoffes wesentlich zu vermindern, und dadurch die Ölproduktion aus der Produktionsbohrung weiter erhöht.

7. Verfahren nach Anspruch 6, worin die Opferverbindung Ligninsulfonat ist.

8. Verfahren nach Anspruch 7, worin die Menge des Ligninsulfonats 0,75 bis 2 Gew.-% beträgt.

## Revendications

1. Un composé répondant à la formule générale:

$$R_1—CH(R_2)—CH_2—O(C_2H_4O)_xCH_2CH_2CH_2SO_3Na$$

dans laquelle $R_1$ est un groupe alkyle ayant 6 à 26 atomes de carbone, $R_2$ est un groupe alkyle ayant 2 à 10 atomes de carbone et x a une value de 2 à 6.

2. Un composé selon la revendication 1, dans lequel $R_1$ a de 8 à 12 atomes de carbone et $R_2$ a de 4 à 6 atomes de carbone.

3. Un procédé de récupération du pètrole d'un réservoir souterrain de pétrole ayant un ou plusieurs puits de production et un ou plusieurs puits d'injection en contact avec le réservoir, comprenant l'injection dans le réservoir de pétrole d'un milieu aqueux qui comprend, comme agent tensio-actif unique, un composé selon la revendication 1 ou la revendication 2 à une concentration efficace pour accroître la production de pétrole par le puits de production au cours de l'injedction secondaire d'eau ou pour récupérer le pètrole tertiaire résiduel lorsque le réservoir de pétrole a été précédemment soumis à une injection.

4. Un procédé selon la revendication 3, dans lequel le réservoir de pétrole est un réservoir à forte salinité.

5. Un procédé selon la revendication 4, dans lequel le réservoir de pétrole a une salinité correspondant à une saumure à 4 à 30%.

6. Un procédé selon l'une quelconque des revendications 3 à 5, dans lequel le milieu aqueux comprend un composé consommable en une quantité efficace pour réduire notablement l'adsorption de l'agent tensio-actif afin d'accroître ainsi la production de pètrole par le puits de production.

7. Un procédé selon la revendication 6, dans lequel le composé consommable est un ligninesulfonate.

8. Un procédé selon la revendication 7, dans lequel la quantité de ligninesulfonate est de 0,75 à 2% en poids.